Europäisches Patentamt

European Patent Office

Office européen des brevets

(19)

(11) Publication number: 0 404 186
A2

# EUROPEAN PATENT APPLICATION

(21) Application number: 90111896.8

(22) Date of filing: 22.06.90

(51) Int. Cl.⁵: G01N 33/68

(30) Priority: 22.06.89 JP 160226/89

(43) Date of publication of application:
27.12.90 Bulletin 90/52

(84) Designated Contracting States:
AT BE CH DE DK ES FR GB GR IT LI LU NL SE

(71) Applicant: DAIICHI PURE CHEMICALS CO. LTD.
13-5, Nihonbashi 3-chome
Chuo-ku Tokyo(JP)

(72) Inventor: Teramura, Yasuo, c/o Daiichi Pure Chem. Co.,Ltd
Tokyo Research Center, 5-5-12, Narihira,
Sumida-ku
Tokyo(JP)
Inventor: Koizumi, Hiroshi, c/o Daiichi Pure Chem. Co.,Ltd
Tokyo Research Center, 5-5-12, Narihira,
Sumida-ku
Tokyo(JP)
Inventor: Xu, Chin-Zhi, c/o Daiichi Pure Chem. Co.,Ltd
Tokyo Research Center, 5-5-12, Narihira,
Sumida-ku
Tokyo(JP)
Inventor: Sakai, Yasuo, c/o Daiichi Pure Chem. Co.,Ltd
Tokyo Research Center, 5-5-12, Narihira,
Sumida-ku
Tokyo(JP)

(74) Representative: Kraus, Walter, Dr. et al
Patentanwälte Kraus, Weisert & Partner
Thomas-Wimmer-Ring 15
D-8000 München 22(DE)

(54) Process of measuring specific antibody.

(57) A process for measuring a specific antibody in a specific class of immunoglobulin contained in a human body fluid sample is disclosed. The process comprises removing immunoglobulins of the classes other than said specific class from said sample and subjecting the sample from which the immunoglobulins of said other classes have been removed to a specific antibody measurement. It can quantitatively measure the target specific antibody by a simple procedure without the interference by other classes of immunoglobulins, giving an accurate determination in a short time.

## PROCESS OF MEASURING SPECIFIC ANTIBODY

## BACKGROUND OF THE INVENTION

Field of the Invention:

The present invention relates to a process for immunologically measuring specific antibodies, and, more particularly, to an immunoassay which ensures accurate quantitative analysis of only the target specific antibody by a simple procedure.

Description of the Background Art:

A remarkable increase in allergic diseases in recent years calls for the need for more exact and reliable diagnosis and treatment of theses diseases. In particular, in the I-type allergy caused by the allergen-specific IgE of which a major cause is antigens (allergens) such as pollen, mites, house dust, or the like, the measurement of the allergen-specific IgE antibody is very important in order to detect the allergen which could be the cause of the allergic disease. A quick and accurate quantitation of specific antibodies is therefore strongly demanded in clinics.

A method involving a cutaneous reaction, the PK test. a method using chopped human lung, and the like are conventionally employed in the determination of allergens. These methods, however, entail a risk of occurence of unfavorable reactions in human bodies such as the anaphylactoid shock and the like. They also have a problem in that the reaction itself becomes unstable during the therapy. Because of these reasons, these methods are seldom used in actual clinics. On the other hand, the radioallergosorbent test (RAST) method developed by Wide et al. [Wide L., *Lancet, 2,* 1105-1107 (1967)] as an *in vitro* serum diagnostic method is widely used for the measurement of specific IgE antibody for an antigen (allergen). A problem in this method is related to the significant differences in concentrations of each class of immunoglobulins in serum as indicated in Table 1. In particular, IgE exhibits a concentration about 10,000 times lower than immunoglobulins of other classes.

TABLE 1

| Class | Normal concentration in serum (mg/ml) |
|-------|---------------------------------------|
| IgG | 12.5 |
| IgA | 2.1 |
| IgM | 1.3 |
| IgE | $3 \times 10^{-4}$ |
| Source: *Immunology 2,* edited by Yuich Yamamura, Nakayama Shoten | |

Because of the above differences in immunoglobulin concentrations, the measurement of specific IgE by the RAST method could greatly be interferes by specific IgG, IgA, or IgM. A number of reports indicated this possibility [e.g *Allergy, 24,* (4), 217-221 (1975); *J. Allergy Clin. Immunol., 67,* (2), 87-89 (1981)]. More recently, the measurement of specific IgG antibody or specific IgA antibody by the BLISA method and the like has been attempted. Since these methods are the same with the IgE-RAST method in principle, the interference by the specific antibodies from the other classes of immunoglobulins has been inevitable, thus posing a serious problem in an accurate determination of specific antibody concentration.

In the specific desensitization therapy which is old in the art as a therapeutic method of allergic diseases, the production of a blocking antibody, i.e. the production of specific IgG antibody, has been revealed to have involvement as a part of the mechanism. More recently, the specific IgG antibody has been indicated to act not only as a blocking antibody but also as an anaphylactoid antibody. Another report indicated that the specific IgA antibody acted to repress the occurrence of allergic diseases. Thus, it has been revealed that all classes of immunoglobulins in serum had some involvements in allergic reactions. Accurate determination of specific antibodies of all classes is therefore very important for the diagnosis and treatment of allergic diseases.

Simple and easy procedure and quick determination are also important factors in the specific antibody measurement for the purpose of the diagnosis and treatment of allergic diseases.

In view of this situation the present inventors have undertaken extensive studies in order to develop a simple and quick measuring method which is free from the above problems, and found that the problem of the interference by the other antibodies could be eliminated by removing in advance the immunoglobulins of the classes other than the class of the immunoglobulin of which the specific antibody is to be determined. The inventors have also found that such removal of the other immunoglobulins from samples ensured a simple procedure which can readily be applied to a daily diagnosis of the diseases. Such findings have led to the completion of the present invention.

## SUMMARY OF THE INVENTION

Accordingly, an object of this invention is to provide a process for measuring a specific antibody in a specific class of immunoglobulin contained in a human body fluid sample which comprises: removing immunoglobulins of the classes other than said specific class from said sample and subjecting the sample from which the immunoglobulins of said other classes have been removed to a specific antibody measurement.

Other objects, features and advantages of the invention will hereinafter become more readily apparent from the following description.

## BRIEF DESCRIPTION OF THE DRAWINGS

Figure 1 shows the dilution curve of the acarid allergen-specific IgE antibody prepared in Example 3.

Figure 2 shows the dilution curve of the albumen allergen-specific IgG antibody prepared in Example 4.

Figure 3 shows the dilution curve of the house dust allergen-specific IgA antibody prepared in Example 5.

## DETAILED DESCRIPTION OF THE INVENTION AND PREFERRED EMBODIMENTS

In the practice of present invention, prior to the determination of the specific antibody, immunoglobulins other than the immunoglobulin of the specific class which is to be measured are removed from a human body fluid which is a sample to be detected (such a specific antibody to be detected and said immunoglobulins to be removed in advance are hereinafter respectively referred to as "subject antibody" and "competitive antibody"). The competitive antibodies are, in principle, (1) IgG, IgA and IgM when the subject antibody is IgE, (2) IgG, IgA and IgE when the subject antibody is IgM, (3) IgG, IgM and IgE when the subject antibody is IgA, and (4) IgA, IgM and IgE when the subject antibody is IgG. Elimination of immunoglobulins which could give a great impact on the subject antibody considering the magnitude of the differences in the immunoglobulin concentrations in serum, i.e., IgG ≫ IgA > IgM ≫ IgE (as can be seen in Table 1), may give a sufficient result.

Among antibodies which can be a subject antibody in the present invention, i.e., among IgE, IgA, IgM, and IgG, the antibodies to which the process of the present invention is suitably applied are IgE, IgA, and IgM, and especially IgE, on which the influence of the competitive antibody is great.

Human body fluid samples which can be used in the present invention include, for example, plasma, serum, pituita, tear, cerebrospinal fluid, and the like.

As a method of removing competitive antibodies from these human body fluid samples, a method of treating the sample with a substance capable of specifically reacting with the competitive antibodies can be used. Such substances include protein A, protein G, recombinant protein A, recombinant protein G, immunoglobulin antibodies (including monoclonal antibodies), immunoglobulin receptors, and the like.

3

These active substances can be used either independently or in combination. Table 2 shows capabilities of protein A and protein G to combine with various immunoglobulins.

TABLE 2

| Immunoglobulin | Protein A | Protein G |
|---|---|---|
| IgG$_1$ | + + | + + |
| IgG$_2$ | + + | + + |
| IgG$_3$ | - | + + |
| IgG$_4$ | + + | + + |
| IgA | + | - |
| IgM | + | - |
| IgE | - | - |

The use of a carrier with which the active substance is bound is desirable for the treatment of a human body fluid sample in order to combine the competitive antibodies with the active substance and to remove them. More specifically, an affinity carrier such as a membrane, e.g. a membrane of nitrocellulose, cellulose acetate, nylon; or a gel, e.g. agarose, sepharose, dextran, polyacrylamide, with which an active substance is bound in advance, is contacted with a human body fluid sample, thereby removing competitive antibodies from the sample.

Bonding of active substances to carriers can be effected, for example, by physical adsorption or by other known methods such as the CNBr method ["Affinity Chromatography and Affinity Label", *Ptotein Nicleic Acid Enzyme*, Supplement Vol. 22 (1980)].

A human body fluid sample from which the competitive antibodies have been removed in the manner mentioned above (such a sample is hereinafter referred to as "competitive antibody-free sample) contains only the same class of immunoglobulin as the subject antibody. It can be served for a quantitative analysis of the antibody according to a conventional immunoassay. Preferable immunoassays are radioimmunoassay ("Radioimmunoassay, New Edition", Kazuo Shizume and Yuichi Kumahara, Asakura Publishing Co.), enzymeimmunoassay ("Enzyme Immunoassay", 3rd Edition, Eiji Ishikawa et al., Igaku Shoin), latex immunonephelometry, particle size distribution latex immunoassay, and the like.

When the subject antibody is allergen-specific IgE antibody, a particularly preferable process is first to react a mixture of an insoluble carrier sensitized with the allergen and anti-IgE specific antibody with a competitive antibody-free sample, and to measure the degree of the agglomeration. A preferred embodiment using this process is described in more detail.

High molecular or polymer carriers such as polyamide, polyvinylidene chloride, polyvinyl chloride, polyacryl, polyvinyl acetate, polystyrene, polypropylene, polyepoxy, polyurethane, polyethylene, agarose, xthantone, pullulan, and the like may be used as an insoluble carrier. It is desirable that insoluble carriers contain a certain proportion of acidic group such as carboxylic acid group and sulfonic acid group. High molecular compounds or polymers used as a carrier can preferably be prepared by the copolymerization of a monomer without an acid group and a monomer having an acid group. Such insoluble carriers are preferably particles of a size between 0.1 to 3 $\mu$m, with especially preferable size being 0.6 to 1.2 $\mu$m.

An allergen with which the insoluble carrier is sensitized may be acarid, house dust, *Arternaria, Candida,* cat hair, milk, egg-white, soybean, Japan ceder pollen, and the like. They can be used as are, or fractions which are extracted and partially purified or allergen-specific substances obtained by further purification can be used for the purpose of the present invention.

Conventional methods can be used for the sensitization of the insoluble carriers with an allergen. An example is a physical adsorption method in which a buffer solution of an allergen is added to a suspension of insoluble carrier in a buffer and the mixture is allowed to stand for a period from 30 minutes to 1 day, followed by removal of unsensitized allergen by a suitable means such as centrifugation. A chemical conjugation method using a two-liquid type cross-linking reagent or other bonding reagent for sensitizing the insoluble carrier with an allergen can also be used. A suitable concentration of allergen used for the sensitization is usually 0.1 to 2% by weight. It is needless to mention that the concentration may vary depending on such factors as the types and characteristics of the allergen, the kinds and particle size of insoluble carrier, the degree of modification of the carrier with acid groups, etc.

The insoluble carrier sensitized with an allergen (hereinafter referred to as "insoluble allergen reagent") can be stored suspended in a buffer or freeze-dried, if required, after treatment with bovine serum albumin

4

(hereinafter referred to as BSA) or various kinds of surfactants.

As an anti-IgE specific antibody, an anti-IgE specific monoclonal antibody or an anti-IgE specific polyclonal antibody which is purified by affinity chromatography can preferably be used. An anti-IgE specific monoclonal antibody can be prepared by a cell fusion or other method using a mouse or the like which have been immunized with human IgE antibody.

The determination of an allergen-specific IgE antibody can be carried out, for example, by adding a competitive antibody-free sample to a mixture of an insoluble allergen reagent and an anti-IgE specific antibody, incubating the mixture, and measuring the degree of the resulting agglomeration.

The incubation can usually be carried out at 20 to 45°C for 10 to 30 minutes. The laser diffusion method, the electric resistance method, or the like can be employed for the detection of agglomerated and non-agglomerated particles. As a means of the determination of the agglomeration degree, the amount of particles corresponding to the insoluble allergen reagent and those corresponding to the agglomerated particles are determined. For example, a calibration curve can be prepared first from the ratio (reaction rate) of the amounts of agglomerated particles and non-agglomerated particles using an allergen-specific IgE antibody reagent having a known antibody concentration, and based on the curve the amount of the allergen-specific IgE antibody in a sample can be determined. A particle measurement device based on the conventional electric resistance method (the Coulter principle) can accurately measure the amount of allergen-specific IgE antibodies according to the process of the present invention, if a 20 to 50 $\mu$m diameter is used for the orifice aperture.

As illustrated above, by using the process of the present invention a target specific antibody can be quantitatively measured by a simple procedure without the interference by other classes of immunoglobulins. In particular, in the determination of allergen-specific IgE antibodies, in addition to the highly sensitive and simple analysis, a surprisingly quick determination, e.g. within 1 hour starting from sampling of blood, is ensured by the present invention. Thus, the process is very useful in clinics and diagnostic investigations.

Other features of the invention will become apparent in the course of the following description of the exemplary embodiments which are given for illustration of the invention and are not intended to be limiting thereof.

## EXAMPLES

Example 1 Preparation of an Affinity Membrane for removing competitive antibodies

(1) Preparation of recombinant protein G-bound membrane

4 mg of recombinant protein G (product of Genex Corp., purchased from Cosmo Bio Co., Ltd.) was dissolved in 50 ml of 25 mM phosphate-physiological saline buffer (pH 7.0; hereinafter referred to as PBS). A 10 x 10 cm Immunodyne Membrane (trade mark: manufactured by Nippon Pole Co., Ltd.; aperture diameter: 0.2 $\mu$m) was immersed into the solution and shaken for 3 hours at room temperature, followed by removal of the resulting reaction solution. The membrane was washed twice with PBS and immersed into 50 ml of a PBS containing 5% (w/v) BSA and 0.05% Tween (BSAPBS). After shaking overnight at room temperature and removal of BSAPBS, the membrane was washed 5 times with 100 ml of 50 mM glycin buffer (pH 8.2; hereinafter referred to as GB). The protein G-bound membrane thus prepared was dried in the air. Diskettes with a 25 mm diameter were cut out from the membrane, installed in a membrane holder (Swineks: trade mark, manufactured by Millipore Co.; diameter 25 mm), and served for the treatment of samples.

(2) Preparation of anti-human IgA antibody-bound membrane

5 mg of anti-human IgA monoclonal antibody (A-01, product of Yamasa Soybean Source Co.) was dissolved into 50 ml of PBS. A 10 x 10 cm Immunodyne Membrane was immersed into the solution and shaken for 3 hours at room temperature, followed by removal of the resulting reaction solution. The membrane was washed twice with PBS and immersed into 50 ml of BSAPBS. After shaking overnight at room temperature and removal of BSAPBS, the membrane was washed 5 times with 100 ml of 50 mM GB. The anti-human IgA antibody-bound membrane thus prepared was dried in the air. Diskettes with a 25 mm diameter were cut out from the membrane, installed in a membrane holder, and served for the treatment of samples.

### (3) Preparation of anti-human IgM antibody-bound membrane

5 mg of anti-human IgM monoclonal antibody (M-01-AS, product of Yamasa Soybean Source Co.) was dissolved into 50 ml of PBS. A 10 x 10 cm Immunodyne Membrane was immersed into the solution and shaken for 3 hours at room temperature, followed by removal of the resulting reaction solution. The membrane was washed twice with PBS and immersed into 50 ml of BSAPBS. After shaking overnight at room temperature and removal of BSAPBS, the membrane was washed 5 times with 100 ml of GB. The anti-human IgM antibody-bound membrane thus prepared was dried in the air. Diskettes with a 25 mm diameter were cut out from the membrane, installed in a membrane holder, and served for the treatment of samples.

Example 2 Preparation of Affinity Gel for removing competitive antibodies

### (1) Preparation of protein G Sepharose

Recombinant protein G was bonded to CNBr-activated Sepharose 4B (trade mark, product of Pharmacia) according to a conventional method. Specifically, 10 mg of protein G was dissolved into 10 ml of 0.1 M sodium bicarbonate buffer containing 0.5 M sodium chloride (pH 8.3; hereinafter referred to as coupling buffer). 5 ml of CNBr-activated Sepharose 4B which had been swelled in 1 mM hydrochloric acid solution and washed thoroughly with the coupling buffer was added to the solution and reacted for 3 hours at room temperature while gently stirring. After removal of the coupling buffer, 0.1 M Tris-HCl buffer (pH 8.0) was added, followed by further stirring at room temperature for 2 hours. The product was washed with 0.1 M acetate buffer containing 0.5 M NaCl (pH 4.0) and 0.1 M Tris-HCl buffer containing 0.5 M NaCl (pH 8.0), and was ultimately suspended into 50 mM glycin buffer (pH 8.2) and stored at 4°C. This protein G-Sepharose can be replaced by Protein G Sepharose 4 Fast Flow (trade mark, product of Pharmacia).

### (2) Preparation of anti-human IgA antibody Sepharose

Anti-human IgA monoclonal antibody was bonded to CNBr-activated Sepharose 4B (trade mark, product of Pharmacia) according to a conventional method. Specifically, 15 mg of anti-human IgA monoclonal antibody was dissolved into 10 ml of 0.1 M sodium bicarbonate buffer containing 0.5 M NaCl (pH 8.3; coupling buffer). 5 ml of CNBr-activated Sepharose 4B which had been swelled in 1 mM HCl solution and washed thoroughly with the coupling buffer was added to the solution and reacted for 3 hours at room temperature while gently stirring. After removal of the coupling buffer, 0.1 M Tris-HCl buffer (pH 8.0) was added, followed by further stirring at room temperature for 2 hours. The product was washed with 0.1 M acetate buffer containing 0.5 M NaCl (pH 4.0) and 0.1 M Tris-HCl buffer containing 0.5 M NaCl (pH 8.0), and was ultimately suspended into 50 mM glycin buffer (pH 8.2) and stored at 4°C.

### (3) Preparation of anti-human IgM antibody Sepharose

Anti-human IgM monoclonal antibody was bonded to CNBractivated Sepharose 4B according to a conventional method. Specifically, 15 mg of anti-human IgM monoclonal antibody was dissolved into 10 ml of 0.1 M sodium bicarbonate buffer containing 0.5 M NaCl (pH 8.3; coupling buffer). 5 ml of CNBr-activated Sepharose 4B which had been swelled in 1 mM HCl solution and washed thoroughly with the coupling buffer was added to the solution and reacted for 3 hours at room temperature while gently stirring. After removal of the coupling buffer, 0.1 M Tris-HCl buffer (pH 8.0) was added, followed by further stirring at room temperature for 2 hours. The product was washed with 0.1 M acetate buffer containing 0.5 M NaCl (pH 4.0) and 0.1 M Tris-HCl buffer containing 0.5 M NaCl (pH 8.0), and was ultimately suspended into 50 mM glycin buffer (pH 8.2) and stored at 4°C.

Example 3 Measurement of Acarid Allergen-Specific IgE Antibody

(1) Sensitization of insoluble carrier with acarid allergen

0.1 ml of 1M disuccinimidyl glutarate (BISHOX: trade mark, product of Inover Chemicals, Inc., purchased from Funakoshi Pharmaceutical Co., Ltd.) dissolved in dimethylsulfoxide (DMSO) was added to 10 ml of 1% polystyrene latex having 1.0 μm particle size (N-47: trade mark, product of Japan Synthetic Rubber Co., Ltd.) which had been suspended in 20 mM phosphate buffer (pH 7.0; PB). The mixture was stirred and allowed to stand for 10 minutes at room temperature, followed by an addition of 10 ml of 0.4 mg/ml acarid allergen solution. After thorough mixing, the solution was allowed to stand for 1 hour at room temperature. To this solution was added 20 ml of 50 mM glycin buffer containing 0.5% (w/v) BSA (pH 8.2), followed by incubation at 37°C for 2 hours. After washing and removal of the allergen solution and the buffer, the product was ultimately suspended into 100 ml of 50 mM glycin buffer containing 0.2% BSA and 0.1% (w/v) sodium azide and stored at 4°C.

(2) Measurement of acarid allergen-specific IgE antibody

(i) 0.5 ml of a sample diluted to a 20-fold volume with 50 mM glycin buffer containing 0.2% BSA and 0.5% NaCl (pH 8.2) was passed though a protein G-bound membrane, anti-human IgA antibody-bound membrane, and anti-human IgM antibody-bound membrane which were installed in layer in a membrane holder.

(ii) 50 μl of the sample, 50 μl of the latex reagent sensitized with acarid allergen which was prepared in (1), and 50 μl of anti-human IgE monoclonal antibody (E-01, product of Yamasa Soybean Source Co. were mixed and incubated at 37°C for 30 minutes. After terminating the reaction by the addition of 3.0 ml of GB, the solution was diluted with an electrolyte (Isoton II: trade mark, manufactured by Coulter Co.) to a 100-fold volume.

(iii) The particle size distribution of the reaction agglomerates in the diluted solution was measured by Coulter counter-ZBI & C-1000 (trade mark, manufactured by Coulter Co.) and the ratio by volume of the non-agglomerated and agglomerated latexes (RV) was calculated by PC9801 (Nippon Denki Co., Ltd.).

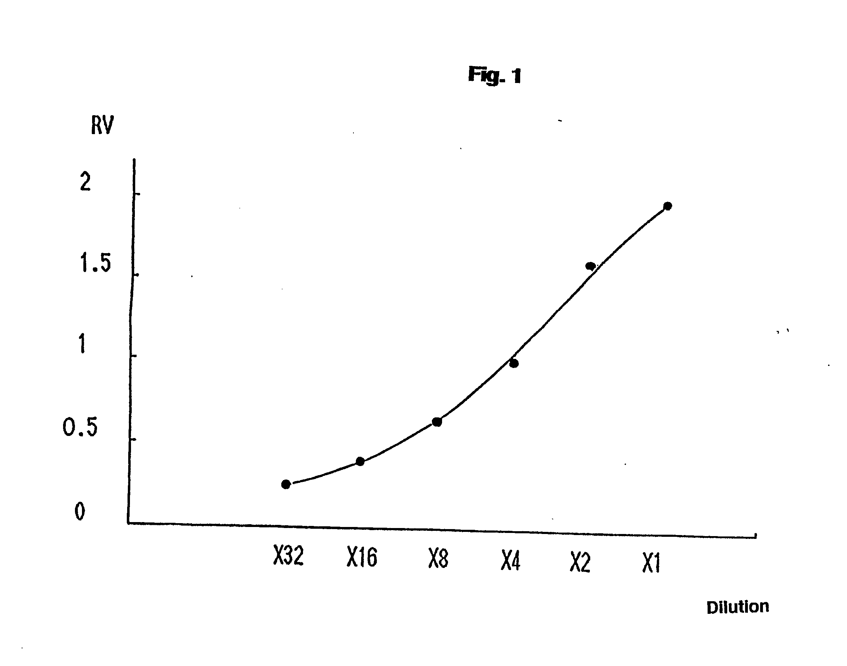

(iv) The dilution curve of the acarid allergen-specific IgE antibody positive sample is shown in Figure 1, in which the rate of dilution is taken along the axis of ordinate and RV is taken along the axis of abscissa.

Example 4 Measurement of Albumen Allergen-Specific IgG Antibody

(1) Sensitization of insoluble carrier with albumen allergen

10 ml of 0.4 mg/ml albumen allergen solution in a glycin buffer (pH 8.2: GB) was added to 10 ml of 1% polystyrene latex having 1.0 μm particle size (SFL140L-10: trade mark, product of Japan Synthetic Rubber Co., Ltd.) which had been suspended in 20 mM GB. The mixture was thoroughly mixed and allowed to stand for 2 hours under ice-cooling, followed by an addition of 20 ml of 50 mM glycin buffer containing 0.5% (w/v) BSA (pH 8.2). The mixture was incubated at 37°C for 4 hours. After washing and removal of the allergen solution and the buffer by centrifugation, the product was ultimately suspended into 100 ml of 50 mM glycin buffer containing 0.2% BSA and 0.1% sodium azide and stored at 4°C.

(2) Measurement of albumen allergen-specific IgG antibody

(i) 0.5 ml of a sample diluted to a 50-fold volume with 50 mM glycin buffer containing 0.2% BSA and 0.5% NaCl (pH 8.2) was passed though anti-human IgA antibody-bound membrane and anti-human IgM antibody -bound membrane which were installed in layer in a membrane holder.

(ii) 50 μl of the sample, 50 μl of the latex reagent sensitized with albumen allergen which was prepared in (1), and 50 μl of 0.1 mg/ml anti-human IgG monoclonal antibody (G-01, product of Yamasa Soybean Source Co.) were mixed and incubated at 37°C for 20 minutes. After terminating the reaction by the addition of 3.0 ml of GB, the solution was diluted with an electrolyte to a 100-fold volume.

(iii) The particle size distribution of the reaction agglomerates in the diluted solution was measured by Coulter counter-ZBI & C-1000 and the ratio by volume of the non-agglomerated and agglomerated latexes (RV) was calculated by PC9801.

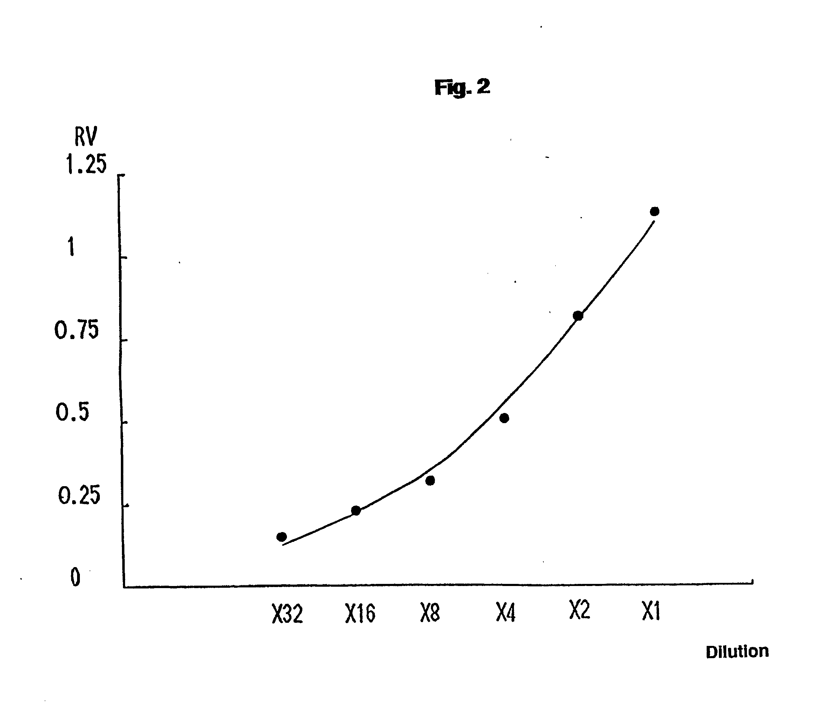

(iv) The dilution curve of the albumen allergen-specific IgG antibody positive sample is shown in Figure 2, in which the rate of dilution is taken along the axis of ordinate and RV is taken along the axis of abscissa.

Example 5 Measurement of House Dust Allergen-Specific IgA Antibody

(1) Sensitization of insoluble carrier with house dust allergen

3 ml of 13.5 mg/ml N-ethyl-N-(3-dimethylaminopropyl)-carbodiimide hydrochloride (BCDI: trade mark, product of BDH Corp.) dissolved in a 20 mM borate buffer (pH 8.2: BB) was added to 10 ml of 1% polystyrene latex having 1.0 μm particle size (L-0101: trade mark, product of Japan Synthetic Rubber Co., Ltd.) which had been suspended in BB. The mixture was stirred, allowed to stand for 1 hour at room temperature, and centrifuged to wash out a surplus amount of BCD1. The product was again suspended in 10 ml of BB, and to the suspension was added 10 ml of 0.5 mg/ml house dust allergen solution. After thorough mixing, the solution was allowed to stand overnight at 4°C. To this solution was added 20 ml of 50 mM glycin buffer containing 0.5% BSA (pH 8.2), followed by incubation at 37°C for 2 hours. After washing and removal of the allergen solution and the buffer by centrifugation, the product was ultimately suspended in 100 ml of 50 mM glycin buffer containing 0.2% BSA and 0.1% sodium azide and stored at 4°C.

(2) Measurement of house dust allergen-specific IgA antibody

(i) 0.1 ml of the sample was added to a miniature column in which 0.4 ml of Protein C Sepharose and 0.3 ml of anti-human IgM antibody sepharose were layered. The column was eluted with 2.0 ml of 50 mM glycin buffer (pH 8.2) containing 0.2% BSA and 0.5% NaCl.

(ii) 50 μl of the sample, 50 μl of the latex reagent sensitized with house dust allergen which was prepared in (1), and 50 μl of 0.1 mg/ml anti-human IgA monoclonal antibody (A-01, product of Yamasa Soybean Source Co.) were mixed and incubated at 37°C for 20 minutes. After terminating the reaction by the addition of 3.0 ml of GB, the solution was diluted with an electrolyte to a 100-fold volume.

(iii) The particle size distribution of the reaction agglomerates in the diluted solution was measured by Coulter counter-ZBI & C-1000 and the ratio by volume of the non-agglomerated and agglomerated latexes (RV) was calculated by PC9801.

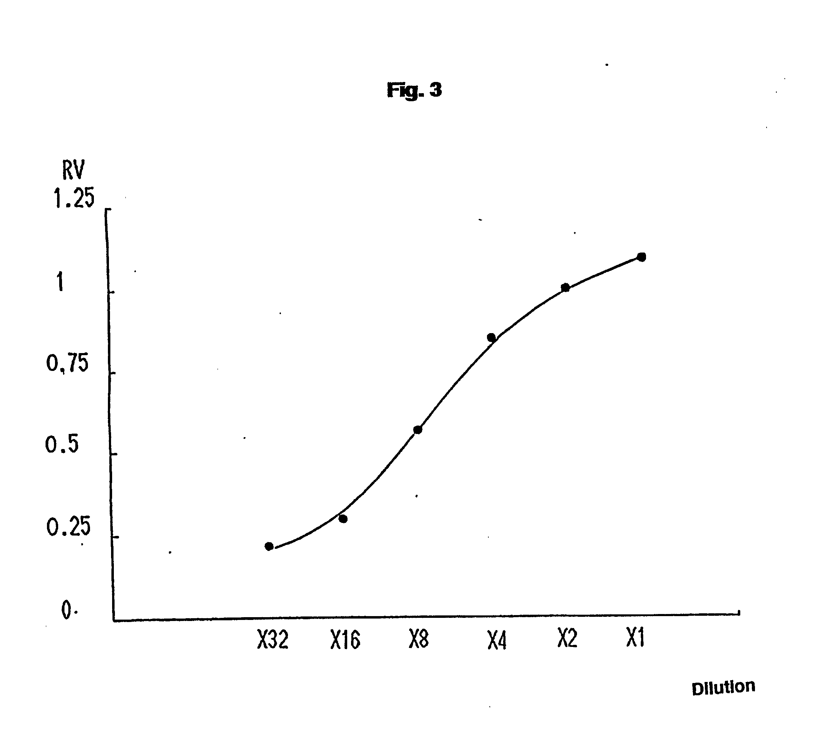

(iv) The dilution curve of the house dust allergen-specific IgA antibody positive sample is shown in Figure 3, in which the rate of dilution is taken along the axis of ordinate and RV is taken along the axis of abscissa.

Obviously, numerous modifications and variations of the present invention are possible in light of the above teachings. It is therefore to be understood that within the scope of the appended claims, the invention may be practiced otherwise than as specifically described herein.

**Claims**

1. A process for measuring a specific antibody in a specific class of immunoglobulin contained in a human body fluid sample which comprises:
removing immunoglobulins of the classes other than said specific class from said sample, and
subjecting the sample from which the immunoglobulins of said other classes have been removed to a specific antibody measurement.

2. A process according to Claim 1, wherein the removal of immunoglobulins of the classes other than the immunoglobulin to be detected is carried out using an active substance which can specifically react with said immunoglobulins to be removed.

3. A process according to Claim 2, wherein said active substance which can specifically react with an

8

immunoglobulin is one or more members selected from the group consisting of protein A, recombinant protein A, protein G, recombinant protein G, immunoglobulin antibodies, and immunoglobulin receptors.

4. A process according to Claim 1, wherein the specific antibody to be measured is an allergen-specific IgE antibody.

5. A process for measuring an allergen-specific IgE antibodies in a human body fluid sample which comprises:

removing IgG, IgA, and IgM from said sample,

reacting a mixture of an insoluble carrier sensitized with an allergen and anti-IgE specific antibody with said sample, and

measuring the degree of agglomeration of the sample.

Fig. 1

EP 0 404 186 A2

## Fig. 2

Fig. 3

RV
1.25
1
0,75
0.5
0.25
0.

X32   X16   X8   X4   X2   X1

Dilution